(19) Europäisches Patentamt
European Patent Office
Office européen des brevets

(11) **EP 1 739 191 A2**

(12) **EUROPEAN PATENT APPLICATION**

(43) Date of publication:
**03.01.2007 Bulletin 2007/01**

(51) Int Cl.:
***C12Q 1/68*** *(2006.01)*

(21) Application number: **06253217.1**

(22) Date of filing: **21.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.07.2005 US 173590**

(71) Applicant: **Agilent Technologies, Inc.**
**Palo Alto, CA 94306 (US)**

(72) Inventors:
• **Wang, Hui**
**Loveland, CO 80537-0599 (US)**
• **Curry, Bo**
**Loveland, CO 80537-0599 (US)**

(74) Representative: **Ellis, Katherine Elizabeth Sleigh et al**
**Williams Powell**
**Morley House**
**26-30 Holborn Viaduct**
**London EC1A 2BP (GB)**

(54) **Melting temperatures matching for producing a probe set**

(57) Aspects of the disclosure are generally directed to probes and probe compositions for detecting or quantifying a target. One aspect provides one or more nucleic acid probes having melting temperatures for their respective nucleic acid targets within less than about 15°C of each other. Another aspect provides arrays including the disclosed probes and methods of using the arrays and the probes. Still another aspect provides methods of making the disclosed probes and arrays.

**FIG. 1**

## Description

This application relates to melting temperature matching and in particular to a method for producing a probe set, a probe set, an array and a method of obtaining a micro RNA (miRNA) profile.

Gene silencing by RNA molecules has been implicated in a wide variety of physiological pathways including pathologies such as cancer and the control of cell proliferation, cell death, fat metabolism, neuronal patterning, modulation of hematopoietic lineage differentiation, and control of leaf and flower development. Accordingly, there is increasing demand for methods for detecting and quantifying RNA molecules involved in gene regulation.

Conventional methods and standards for detecting and quantifying the presence of RNA molecules employ technologies such as gene chips or arrays. The presence of a specific RNA molecule can be detected when the target RNA molecule binds to a complementary sequence on an array. However, small RNA molecules including miRNA, tiny non-coding RNA (tncRNA), short interfering RNA (siRNA) and small modulatory RNA (smRNA) are difficult to detect because of their small size, low copy number, and susceptibility to enzymatic degradation. The number of copies of a target RNA present in a sample can be minuscule compared to the presence of other transcripts and the target may be undetectable in the presence of overwhelming signal from abundant non-target RNAs.

To discriminate between target and non-target RNA using an array, hybridization must be carried out under conditions in which the target RNA forms a stable duplex with the probes and competing sequences do not form stable duplexes with the probes. Because all probes on an array are typically hybridized at the same temperature, the melting temperatures of probes for target RNAs should be closely matched. Algorithms have been developed to calculate melting temperatures of probes of different lengths for targets in solution, but these algorithms generally produce melting temperature estimates that are not consistent with empirically determined melting temperatures for targets bound to tethered probes on an array. Therefore, current technologies available for the production of probes and detection devices for distinguishing and identifying RNA targets are cumbersome and often unreliable.

According to a first aspect of the present invention there is provided a method for producing a probe set as specified in claim 1.

According to a second aspect of the present invention there is provided a probe set as specified in claim 8.

According to a third aspect of the present invention there is provided an array as specified in claim 9.

According to a fourth aspect of the present invention there is provided a method of obtaining an miRNA profile of a sample as specified in claim 10

Aspects of the disclosure are generally directed to probes and probe compositions for detecting or quantifying a target. One aspect provides one or more nucleic acid probes having melting temperatures for their respective nucleic acid targets within less than about 15°C of each other. Another aspect provides arrays including the disclosed probes and methods of using the arrays and the probes. Still another aspect provides methods of making the disclosed probes and arrays.

Yet another aspect provides a method for producing and designing probes for a target by removing or replacing base-pairing monomers from a complement of the target to produce one or more probes and selecting the probes having a melting temperature for the target in a predetermined range.

Another aspect relates to a computer program product for use with a method and apparatus such as described herein. The program product includes a computer readable storage medium having a computer program stored thereon and which, when loaded into a programmable processor, provides instructions to the processor of that apparatus such that it will execute the procedures required of it to perform a method of the present disclosure.

Preferred embodiments of the present invention are described below, by way of example only, with reference to the accompanying drawings, in which:

Figure 1 schematically illustrates an exemplary method for producing probes having melting temperatures within about 15° C; and
Figure 2 illustrates an exemplary array according to an embodiment of the disclosure.

## Definitions

In the present application, unless a contrary intention appears, the following terms refer to the indicated characteristics.

As used herein the phrase "small RNA" refers to an RNA including no more than about 100 nucleotides (e.g., no more than about 50 nucleotides, or no more than about 30 nucleotides). Small RNAs include: microRNA (miRNA), tiny non-coding RNA (tncRNA), short interfering RNA (siRNA), small modulatory RNA (smRNA), and the like, and mixtures thereof. Small RNA are smaller than messenger RNA. Messenger RNA generally contains many hundreds or thousands or nucleotides. In certain situations, many small RNAs can bind to a single molecule of messenger RNA.

As used herein, the term "microRNA" or "miRNA" refers to a class of small noncoding RNAs. miRNA has been observed to contain, for example, about 20 to about 30 nucleotides (nt). miRNAs are single-stranded RNAs that can be produced from hairpin containing RNA molecules.

As used herein, the term "nucleic acid" refers to a polymer made up of nucleotides, e.g., deoxyribonucleotides or ribonucleotides.

As used herein, the terms "ribonucleic acid" and "RNA" refers to a polymer that includes at least one ribonucleotide, e.g., a polymer made up completely of ribonucleotides.

As used herein, the terms "deoxyribonucleic acid" and "DNA" refers to a polymer made up of deoxyribonucleotides.

As used herein, the term "polynucleotide" includes a nucleotide multimer having any number of nucleotides (for example 10 to 200, or more). This includes polynucleotides in which the conventional backbone has been replaced with a non-naturally occurring or synthetic backbone, as well as polynucleotides containing synthetic or non-naturally occurring nucleotides in which one or more of the conventional or canonical bases has been replaced with a group (natural or synthetic) capable of participating in Watson-Crick type hydrogen bonding interactions. Polynucleotides include single or multiple stranded configurations, where one or more of the strands may or may not be completely aligned with another. For example, a polynucleotide can include DNA (including cDNA), RNA, oligonucleotides, and PNA and other polynucleotides as described in US 5,948,902 and references cited therein (all of which are incorporated herein by reference), regardless of the source.

A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.

An "oligonucleotide" generally refers to a nucleotide multimer of about 10 to 100 nucleotides in length.

As used herein, the phrases "percent (%) nucleic acid sequence identity" and "% nucleic acid sequence identity" used with respect to the target and the disclosed probes and arrays, or the complement thereof refers to the percentage of nucleotides in a sequence of interest or target that are identical with the nucleotides in the disclosed probes and arrays, or the complement thereof after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms. Sequence comparison programs including the publicly available NCBI-BLAST2 (Altschul et al., Nucleic Acids Res. 25:3389-3402 (1997)) can be employed for calculating % identity with its search parameters set to default values.

As used herein, the phrase "stringent conditions" or "high stringency conditions" for hybridization of oligo or polynucleotides include, for example: washing at low ionic strength and high temperature, e.g., 0.015 M sodium chloride/ 0.0015 M sodium citrate/0.1% sodium dodecyl sulfate at 50 °C; hybridization in the presence of a denaturing agent, e.g., 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42 °C; or hybridizing in 50% formamide, 5xSSC (0.75 M NaCl, 0.075 M sodium citrate), 50 mM sodium phosphate (pH 6.8), 0.1% sodium pyrophosphate, 5xDenhardt's solution, sonicated salmon sperm DNA (50 g/ml), an random sequenced 25-mer as blocker, 0.1% SDS, and 10% dextran sulfate at 42 °C and washing at 42 °C in 0.2xSSC (sodium chloride/sodium citrate) and 50% formamide followed by washing with 0.1xSSC containing EDTA at 55 °C.

"Hybridizing" and "binding", with respect to polynucleotides, are used interchangeably. "Hybridizing conditions" for a polynucleotide array refer to suitable conditions of time, temperature and the like, such that a target sequence present in solution will bind to an array feature carrying a complementary sequence to a greater extent than to features carrying only sequences which are not complementary to the target sequence (and preferably at least 20% or 100%, or even 200 or 500% greater).

As used herein, the term "isolated" polynucleotide refers to a polynucleotide that is identified and separated from at least one component of its natural environment. A recombinantly produced or synthetic polynucleotide is an isolated polynucleotide.

As used herein, the phrase "control sequences" refers to DNA sequences controlling transcription of an operably linked polynucleotide in a selected host cell or organism.

As used herein, the phrase "operably linked" refers to a polynucleotide in a functional relationship with another polynucleotide.

An "array", unless a contrary intention appears, includes any one-, two- or three-dimensional arrangement of addressable regions bearing a particular chemical moiety or moieties (for example, biopolymers such as polynucleotide sequences) associated with that region. An array is "addressable" in that it has multiple regions of different moieties (for example, different polynucleotide sequences) such that a region (a "feature" or "spot" of the array) at a particular predetermined location (an "address") on the array will detect a particular target or class of targets (although a feature may incidentally detect non-targets of that feature). Array features can be, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probes" may be the one which is to be evaluated by the other (thus, either one could be an unknown mixture of polynucleotides to be evaluated by binding with the other). Target probes may be covalently bound to a surface of a non-porous or porous substrate either directly or through a linker molecule, or may be adsorbed to a surface using intermediate layers (such as polylysine) or porous substrates.

An "array layout" refers to one or more characteristics of the array or the features on it. Such characteristics include one or more of: feature positioning on the substrate; one or more feature dimensions; some indication of an identity or function (for example, chemical or biological) of a moiety at a given location; how the array should be handled (for example, conditions under which the array is exposed to a sample, or array reading specifications or controls following sample exposure).

A "pulse jet" is a device which can dispense drops in the formation of an array. Pulse jets operate by delivering a pulse of pressure to liquid adjacent an outlet or orifice such that a drop will be dispensed therefrom (for example, by a piezoelectric or thermoelectric element positioned in a same chamber as the orifice).

An array "package" may be the array plus only a substrate on which the array is deposited, although the package may include other features (such as a housing with a chamber).

An "aptamer" generally refers to a double stranded DNA or single stranded RNA molecule that binds to specific molecular targets, such as a protein or metabolite.

A "chamber" references an enclosed volume (although a chamber may be accessible through one or more ports).

A "region" refers to any finite area, for example a finite area on the array that can be illuminated and any resulting fluorescence therefrom simultaneously (or shortly thereafter) detected, for example a pixel.

A "processor" references any hardware and/or software combination which will perform the functions required of it. For example, any processor herein may be a programmable digital microprocessor such as available in the form of a mainframe, server, or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic or optical disk may carry the programming, and can be read by a suitable disk reader communicating with each processor at its corresponding station.

It will also be appreciated that throughout the present application, that words such as "top", "upper", and "lower" are used in a relative sense only.

When one item is indicated as being "remote" from another, this is referenced that the two items are at least in different buildings, and may be at least one mile, ten miles, or at least one hundred miles apart. "Communicating" information references transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

Reference to a singular item, includes the possibility that there are plural of the same items present.

"May" means optionally.

Methods recited herein may be carried out in any order of the recited events which is logically possible, as well as the recited order of events.

All patents and other references cited in this application, are incorporated into this application by reference where permissible except insofar as they may conflict with those of the present application (in which case the present application prevails).

**Method of Producing Probes with Defined Melting Temperatures**

One embodiment, among others, provides a method for producing probes specific for one or more targets wherein the probes have a melting temperature for their respective targets in a range of about 15°C, within about 10°C, or within about 5°C. In certain embodiments, the difference between the maximum and minimum melting temperatures is less than about 20°C, less than about 15°C, less than about 10°C, or less than about 5°C. In another embodiment, the maximum and minimum melting temperatures are the same.

Generally, one or more probes optionally having different sequences of monomers or a different number of monomers are selected based on their melting temperatures for their respective targets, under specified conditions of hybridization. In one embodiment, the probes have melting points for their targets in the range of about 50° to about 60° C, and the specified hybridization conditions are 750 mM salt in the absence of organic modifiers (e.g. formamide). Probes specific for the same target can have different sequences as well as different lengths, but selected probes will each have a melting temperature for their respective targets within about 15° C, within about 10° C, or within about 5°

C of each other. Probes specific for different targets also can have different sequences and different lengths provided they each have a melting temperature for their respective targets of within about 15° C, within about 10° C, or within about 5° C of each other. The probes are generally DNA, but can also include RNA, or can be a combination of RNA and DNA. The targets or probes are naturally occurring or non-naturally occurring polymers including, but not limited to nucleic acids such as RNA, DNA, PNA, polypeptides, and combinations thereof.

Another embodiment provides probes having melting temperatures within about 15° C of each other and wherein the probes hybridize in a sequence dependent manner with an RNA target. Exemplary RNA targets include, but are not limited to, microRNA (miRNA), tiny non-coding RNA (tncRNA), short interfering RNA (siRNA) small modulatory RNA (smRNA), and messenger RNA (mRNA).

In an embodiment, an array includes probes and every one of the probes has a melting temperature for their respective target within about 15°C, within about 10°C, or within about 5°C of each other. In an embodiment, all of the probes in the array have melting points for their targets in the range of about 50° to about 60° C. In an embodiment, an array includes probes and 80% of the probes have a melting temperature for their respective target within about 15°C, within about 10°C, or within about 5°C of each other. In an embodiment, 80% of the probes in the array have melting points for their targets in the range of about 50° to about 60° C.

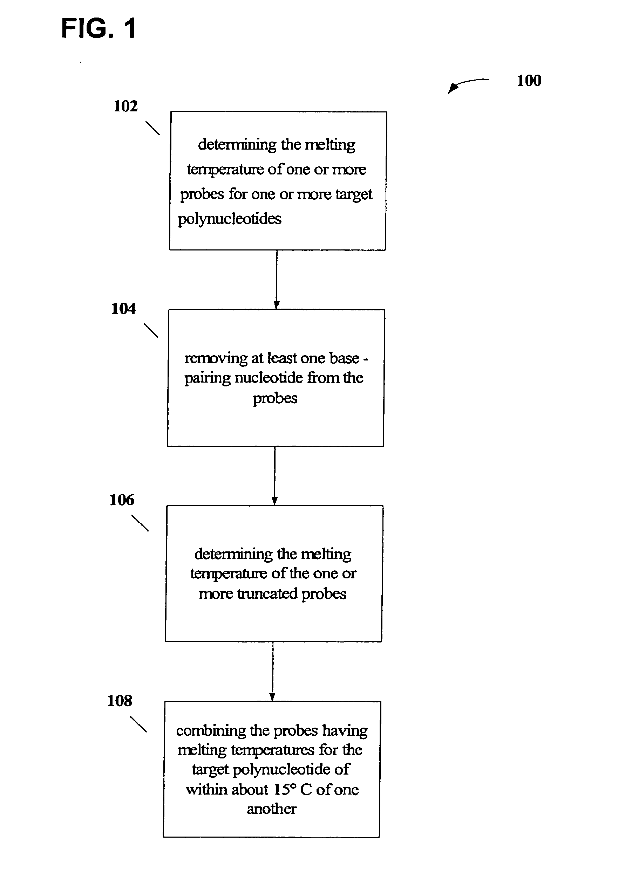

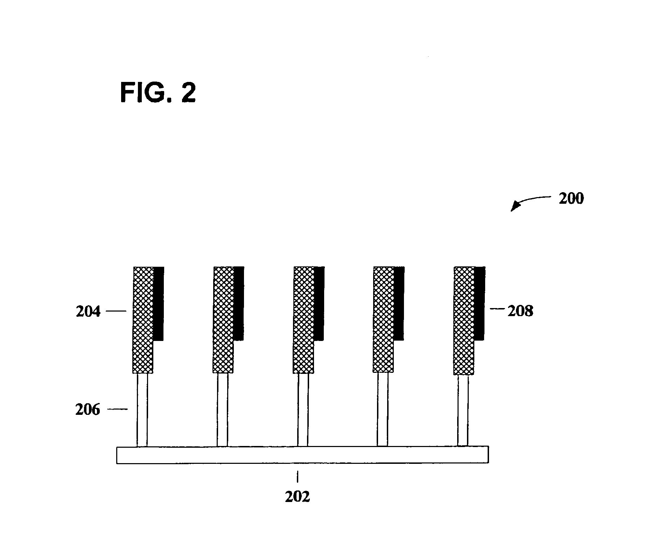

Figures 1 and 2 schematically illustrate two embodiments of the present disclosure. Figure 1 schematically illustrates an exemplary method 100 for producing one or more probes specific for a target, for example a nucleic acid target. The probes contain the complementary sequence to the target as well as complements to any additional nucleotides that may have been added to the targets during pre-hybridization processing. Representative nucleic acid targets include, but are not limited to, microRNA (miRNA), tiny non-coding RNA (tncRNA), short interfering RNA (siRNA) small modulatory RNA (smRNA), fragments thereof, and combinations thereof. In certain embodiments, probes having different lengths but melting temperatures of within less than about 15°C of each other are selected.

Process **100** begins with determining the melting temperature of one or more probes for one or more targets in step **102.** Representative probes are polymers including, but not limited to polymers of a single monomer (homopolymers) or polymers of more than one monomer (heteropolymers). Monomers can be non-naturally occurring or naturally occurring monomers such as deoxyribonucleotides, ribonucleotides, amino acids, sugars, organic molecules capable of base stacking or derivatives thereof.

In one embodiment, a DNA probe complementary to a known target miRNA sequence is used as the initial reference probe. The initial reference probe can be a full length complement or can have at least one region that specifically base pairs with the target miRNA that causes the probe to specifically hybridize to the target miRNA. In another embodiment, the initial reference probe can be a full length complement to the target miRNAs and any additional nucleotides added to the target miRNA which lengthen the miRNA hybridizing sequence. The initial reference probe has a melting temperature for the target represented by "$T_i$" and a number of nucleotide monomers represented by "N". The initial probe also has a number of base pairing nucleotide monomers represented by "X", wherein $N \geq X$.

In step **104,** one or more base-pairing nucleotides of the probe are removed ("Y") or substituted with a non-base pairing nucleotide so that the melting temperature of the probe ($T_p$) is less than $T_i$ and wherein $X > Y$. In certain embodiments 1, 2, 3, 4, 5, 6, 7, 8, 9, 10, 11, 12, 13, 14, 15 or more base-pairing monomers are removed from the initial probe, for example, about 1-8 base-pairing monomers are removed. Probes can be selected to have a melting temperature equal to or less than a $T_i$ and equal to or greater than a minimum predetermined or pre-selected melting temperature $T_{min}$. Generally $T_i$-$T_{min} \leq$ about 15 °C, for example, $\leq$ 10 °C. The base pairing nucleotides of the probes can be removed serially, individually, or in groups to obtain a set of probes that specifically hybridize to a target, wherein at least two probes in the set have different melting temperatures from each other, and wherein at least one of the probes of the set has a melting temperature less than the melting temperature of the initial probe. In another embodiment, the set of probes includes probes having a different number of total nucleotides, a different number of base pairing nucleotides, the same number of nucleotides but with a different number of base-pairing nucleotides, or any combination thereof.

In a particular embodiment, one or more base-pairing nucleotides from the 3' end of the probe are removed or replaced with non-base-pairing nucleotides. For example nucleotides that base-pair with nucleotides 1-8 of the target miRNA are removed or replaced. As the base-pairing nucleotides are removed, the $T_m$ will decrease. Base-pairing nucleotides can be replaced with nucleotides or monomers to reduce or increase the amount of hydrogen bonding between the modified probe and the target so that the modified probe has a melting temperature for the target in a predetermined range.

In step **106,** the $T_m$ of the modified probes are determined either in silico, empirically, or by both methods. Hybridization conditions between the modified probes and the target can optionally be varied so that melting temperatures under various conditions can be obtained. Probes having a $T_m$ in a predetermined range under predetermined hybridization conditions, for example a melting temperature in the range of about 30°C to about 100°C, 40°C to 90°C, 45°C to 85°C, or about 50°C to about 60°C are selected in step **108.** Hybridization conditions include stringency conditions such as salt or ion concentration, concentration of surfactants, concentration of blocking agents such as albumin, time of incubations or washes, and concentration of other buffer components such as formamide, urea, or any combination thereof. In one embodiment, probes having a $T_m$ within about 10° to 15°C of each other are selected. In some embodiments, only truncated probes or substituted probes are selected; whereas, in other embodiments, any combination of full length probes, probes with substituted nucleotides, or truncated probes are selected.

Once the desired probe sequences have been selected, the probes can be synthesized on a solid support or otherwise tethered to a solid support and the $T_m$ for each probe or each class of probes determined empirically. As noted above, the hybridization conditions can be varied. The probes are then reselected according to empirically determined $T_m$ in sets of probes having empirically determined $T_m$s of within about 15°C, within about 10°C, or within about 3°C of each other, for example, for the chosen hybridization condition. These re-selected probes are then incorporated into an array. Generally, the re-selected probes will have $T_m$s of less than the $T_m$ of a probe with its entire length 100% complementary to the target (i.e., the probe is 100% complementary to the target in the binding region). In one embodiment, the selected probes can distinguish between targets such as miRNAs having less than 5, less than 3, or about 1 nucleotide difference in sequence.

One of skill in the art will appreciate that the selected probes can have one or more regions complementary to the same or different targets. Alternatively, the probes can be complementary to different regions of the same target.

## RNA and Melting Temperatures

In one embodiment, the miRNAs which form complexes with the disclosed probes differ from siRNA. For example, miRNAs are derived from genome loci distinct from other recognized sequences; whereas siRNA are derived from mRNAs, transposons, viruses, or heterochromatic DNA. Moreover, miRNAs according to certain embodiments of the disclosure are derived from precursors including a hairpin loop structure. siRNAs are generally processed from long biomolecular RNA duplexes or chemically synthesized. Finally, miRNA/RISC are involved in silencing loci unrelated to that from which it originated. siRNAs, however, are involved in auto-silencing or silencing of the same or similar loci from which they originated.

Another embodiment provides methods and compositions for detecting miRNAs from mammals such as mice and humans, insects such as *Drosophila,* nematodes such as *C. elegans,* and plants such as *Arabidopsis.* Exemplary sequences of miRNA that can be detected and used to produce the disclosed probes include those sequences described in the miRNA Registry published on the internet at http://www.sanger.ac.uk/Software/Rfam/mirna/index.shtml, (See also, Griffiths-Jones S., *The microRNA Registry.* NAR, 2004, 32, Database Issue, D109-D111) both of which are incorporated by reference in their entirety where permissible. Representative miRNA probe sequences include those provided in Table 1. Target sequences include those provided in Table 1 as well as human miRNA genes designated hsa-mir-1 through hsa-mir-450 including for example, hsa-mir-1-1, hsa-mir-1-2, hsa-mir-7, hsa-mir-17, etc., combinations thereof, and fragments thereof.

In certain embodiments, the disclosed probes can be integrated into a diagnostic device, for example an array. The device can be interrogated with a sample to detect the presence and/or amount of one or more targets in the sample. The target or amount of target can then be correlated with a phenotype, for example, propensity to develop a pathology such as cancer. The presence, absence, or quantity of a target or combination of targets can be indicative of a phenotype of the source organism of the sample. More particularly, the disclosed device can be used to detect one or more targets present in a fluid sample including, but not limited to tissue samples, blood, serum, plasma, saliva, sweat, tears, mucous, stool, lymphatic fluid, semen, interstitial fluid, gastric fluid, spinal fluid, or a particular cell, cell type, a plurality of cell types, organism or individual, species, genus, order, or the like. For example, the probes can detect the presence or expression level of a gene or gene transcript or a polypeptide. The target can be found in a particular cell, microorganism, virus, plant, fungus, eukaryote, prokaryote, or other organism.

In certain embodiments, the disclosed probes and arrays can be diagnostic of a particular growth condition, environmental condition, developmental stage, or the like.

In certain embodiments, the disclosed probes and arrays can be diagnostic of a particular subset of inhibitory nucleic acids such as miRNA.

The transition of double-stranded to single-stranded conformation can be monitored as an increase in absorbance and is marked by a sharp increase in the ultraviolet light absorbance by the nucleic acid sample at the temperature where the conformational transition takes place. The temperature corresponding to the midpoint of the absorbance rise is called the melting temperature ($T_m$). In structural terms, $T_m$ is the temperature at which 50% of the base pairs in the duplex have been denatured. Methods for determining the melting temperature of nucleic acid duplexes are known in the art. See for example, Sambrook and Russell (2001) Molecular Cloning: A Laboratory Handbook, 10.38-10.41 and 10.47, which is incorporate by reference in its entirety.

$T_m$ can be determined mathematically using equations and algorithms known in the art. For duplex oligonucleotides shorter than 25 bp, "The Wallace Rule" can be used in which:

$$T_m \text{ (in } °C) = 2(A+T) + 4(C+G),$$

where
(A+T) - the sum of the A and T residues in the oligonucleotide,
(C+G) - the sum of G and C residues in the oligonucleotide
(Wallace, R.B., et al., Hybridization of synthetic oligodeoxyribonucleotides to phiX174 DNA: the effect of single base pair mismatch, Nucleic Acids Res., 6, 3543-3557, 1979). More sophisticated computer programs for estimating $T_m$ are also available (Nicolas Le Novere (2001), MELTING, computing the melting temperature of nucleic acid duplex. Bioinformatics 17(12), 1226-1227). VisualOmp (DNA Software, Inc., Ann Arbor, MI) is a commercially available software program that can be used to estimate initial melting temperatures for probe:target duplexes. Alternatively, $T_m$s can be calculated by extracting parameters from various published thermodynamic studies.

$T_m$ can also be determined empirically using methods known in the art (Sambrook and Russell (2001) Molecular Cloning: A Laboratory Handbook, 10.38-10.41).

Table 1 shows calculated and observed melting temperatures for various miRNA genes and their respective probes. The calculated melting temperature values were obtained using established thermodynamic values from published data. Observed melting temperatures were determined using conventional methods described above or known in the art. Various algorithms and parameters used resulted in melting temperatures that differed significantly from observed melting temperatures.

Table 2 shows the calculated melting temperature for probes and their targets using the software programs VisualOmp and calculation derived from published thermodynamic parameters compared to the empirically determined melting temperatures. The empirically determined melting temperatures in Table 2 were extrapolated from sequential repeated microarray hybridizations similar to the procedure described above. The level of hybridization was determined by the retention of fluorescently labeled synthetic miRNAs by the unlabeled probes attached to the microarray surface. To estimate the melting temperature, the labeled synthetic miRNAs were hybridized to the microarray probes first at 50°C overnight. The washed microarray was scanned to quantify the retained fluorescence. The scanned microarray was then incubated at 50°C for 1.5 hour with hybridizing buffer in the presence of random unlabeled oligonucleotides, but without additional target, and rescanned to quantify fluorescence.

| TABLE 1 Tm (°C) Calculations for DNA/DNA only | | | | | | | | | |
|---|---|---|---|---|---|---|---|---|---|
| **Probe** | **miRNA** | **Probe sequence** | **#NT** | **Probe-Gene** | **Tm Calc** | **Tm Calc** | **Tm Calc** | **Tm Calc** | **Tm Obs** |
| 3-S-D-M | dme-miR-2b | TCCTCAAAGCTGGCTGTG (SEQ ID NO:1) | 18 | 3-S-D-M - dme-miR-2b | 51 | 55 | 58 | 62 | 62 |
| 3-S-R-M | dme-miR-2b | TCCTCAAAGCTGGCTGT (SEQ ID NO:2) | 17 | 3-S-R-M - dme-miR-2b | 48 | 52 | 56 | 60 | 58 |
| 3-G-D*-M | dme-miR-2b | GGCTCCTCAAAGCTGGCTG (SEQ ID NO:3) | 19 | 3-G-D*-M - dme-miR-2b | 57 | 61 | 64 | 68 | 62 |
| 4-S-DR-M | dme-miR-3 | ACACACTTTGCCCAGTGA (SEQ ID NO:4) | 18 | 4-S-DR-M - dme-miR-3 | 50 | 54 | 58 | 62 | 60 |
| 4-G-D*-M | dme-miR-3 | GTGAGACACACTTTGCCCA (SEQ ID NO:5) | 19 | 4-G-D*-M - dme-miR-3 | 53 | 57 | 60 | 64 | 62 |
| 7-S-DR-M | dme-miR-6 | AAAGAACAGCCACTGTGATA (SEQ ID NO:6) | 20 | 7-S-DR-M - dme-miR-6 | 49 | 53 | 56 | 60 | 57 |
| 7-G-D*-M | dme-miR-6 | GAAAAGAACAGCCACTGTG (SEQ ID NO:7) | 20 | 7-G-D*-M - dme-miR-6 | 52 | 55 | 59 | 62 | 57 |
| 16-S-DR-M | dme-miR-14 | TAGGAGAGAGAAAAGACTGA (SEQ ID NO:8) | 21 | 16-S-DR-M - dme-miR-14 | 48 | 51 | 55 | 58 | 52 |
| 16-G-D-M | dme-miR-14 | GTAGGAGAGAGAAAAGACTGA (SEQ ID NO:9) | 22 | 16-G-D-M - dme-miR-14 | 52 | 55 | 58 | 61 | 52 |
| 18-S-DR-M | dme-miR-184* | GGGCGAGAGAATGATAAGG (SEQ ID NO:10) | 19 | 18-S-DR-M - dme-miR-184* | 51 | 54 | 58 | 62 | 56 |
| 18-G-D*-M | dme-mir-184* | GCGGGGCGAGAGAATGATA (SEQ ID NO:11) | 19 | 18-G-D*-M - dme-miR-184* | 55 | 58 | 62 | 65 | 57 |
| 44-S-D-M | dme-miR-285 | ACTGATTTCGAATGGTGCT (SEQ ID NO:12) | 19 | 44-S-D-M - dme-miR-285 | 50 | 53 | 57 | 61 | 56 |
| 44-S-R-M | dme-miR-285 | ACTGATTTCGAATGGTGCTA (SEQ ID NO: 13) | 20 | 44-S-R-M - dme-miR-285 | 50 | 53 | 56 | 60 | 56 |
| 44-G-D*-M | dme-miR-285 | GGCACTGATTTCGAATGGTG (SEQ ID NO:14) | 20 | 44-G-D*-M - dme-miR-285 | 54 | 58 | 61 | 64 | 62 |

| TABLE 1 Tm (°C) Calculations for DNA/DNA only | | | | | | | | | |
|---|---|---|---|---|---|---|---|---|---|
| Probe | miRNA | Probe sequence | #NT | Probe-Gene | Tm Calc | Tm Calc | Tm Calc | Tm Calc | Tm Obs |
| 62-S-DR-M | dme-let-7 | ACTATACAACCTACTACCTCA (SEQ ID NO:15) | 21 | 62-S-DR-M - dme-let-7 | 47 | 50 | 54 | 57 | 62 |
| 62-G-D-M | dme-let-7 | GACTATACAACCTACTACCTCA (SEQ ID NO:16) | 22 | 62-G-D-M - dme-let-7 | 50 | 53 | 56 | 59 | 62 |
| 65-S-DR-M | dme-miR-308 | CTCACAGTATAATCCTGTGATT (SEQ ID NO:17) | 22 | 65-S-DR-M - dme-miR-308 | 49 | 53 | 56 | 59 | 56 |
| 65-G-D*-M | dme-miR-308 | GCTCACAGTATAATCCTGTGATT (SEQ ID NO:18) | 23 | 65-G-D*-M - dme-miR-308 | 53 | 56 | 59 | 63 | 58 |
| 66-S-DR-M | dme-miR-31a | TCAGCTATGCCGACATCTT (SEQ ID NO:19) | 19 | 66-S-DR-M - dme-miR-31a | 50 | 54 | 57 | 61 | 59 |
| 66-G-D*-M | dme-miR-31a | GTCAGCTATGCCGACATCTT (SEQ ID NO:20) | 20 | 66-G-D*-M - dme-miR-31a | 54 | 57 | 60 | 64 | 62 |
| 74-S-D-M | dme-miR-316 | CCAGTAAGCGGAAAAAGAC (SEQ ID NO:21) | 19 | 74-S-D-M - dme-miR-316 | 50 | 54 | 57 | 61 | 50 |
| 74-S-R-M | dme-miR-316 | CCAGTAAGCGGAAAAAGACA (SEQ ID NO:22) | 20 | 74-S-R-M - dme-miR-316 | 52 | 55 | 59 | 62 | 52 |
| 74-G-D*-M | dme-miR-316 | GCGCCAGTAAGCGGAAAAA (SEQ ID NO:23) | 19 | 74-G-D*-M - dme-miR-316 | 55 | 58 | 61 | 65 | 55 |

| TABLE 2 DNA Software DNA-RNA Duplex Tm | | | | |
|---|---|---|---|---|
| Probe | miRNA | Tm VisualOmp | Tm Calculated | Tm Obs |
| 3-S-D-M | dme-miR-2b | 55 | 51.99 | 62 |
| 3-S-R-M | dme-miR-2b | 60 | | 58 |
| 3-G-D*-M | dme-miR-2b | | 58.01 | 62 |
| 4-S-DR-M | dme-miR-3 | 58 | 51.49 | 60 |
| 4-G-D*-M | dme-miR-3 | | 54.08 | 62 |
| 7-S-DR-M | dme-miR-6 | 50 | 50.39 | 57 |
| 7-G-D*-M | dme-miR-6 | 48 | 53.01 | 57 |
| 16-S-DR-M | dme-miR-14 | 45 | 49.17 | 52 |
| 16-G-D-M | dme-miR-14 | 49 | 52.53 | 52 |
| 18-S-DR-M | dme-miR-184* | 51 | 51.67 | 56 |
| 18-G-D*-M | dme-miR-184* | | 55.63 | 57 |
| 44-S-D-M | dme-miR-285 | 54 | 51.03 | 56 |
| 44-S-R-M | dme-miR-285 | | 50.82 | 56 |
| 44-G-D*-M | dme-miR-285 | | 55.24 | 62 |
| 62-S-DR-M | dme-let-7 | 55 | 47.96 | 62 |
| 62-G-D-M | dme-let-7 | 57 | 50.5 | 62 |
| 65-S-DR-M | dme-miR-308 | 64 | 50.4 | 56 |
| 65-G-D*-M | dme-miR-308 | 69 | 54.23 | 58 |
| 66-S-DR-M | dme-miR-31a | 59 | 51.11 | 59 |
| 66-G-D*-M | dme-miR-31a | | 54.54 | 62 |
| 74-S-D-M | dme-miR-316 | 49 | 51.28 | 50 |
| 74-S-R-M | dme-miR-316 | 46 | 52.9 | 52 |
| 74-G-D*-M | dme-miR-316 | 55 | 55.8 | 55 |

This step was then repeated at 55°C for 1.5 hour, and 60°C for 1.5 hour. Since the $T_m$ estimate reported in Table 2 is based on an experiment limited to 50-60°C, the probes reported to have observed $T_m$ of 62°C actually melt at a temperature greater than or equal to 60°C, the highest temperature observed. Nonetheless, it is clear that the observed melting temperatures and the calculated temperatures differ significantly.

The Tm determination protocol described here is one example of empirical Tm determination. In some embodiments, any or all of the incubation time, temperature, solution conditions, sample concentrations, and non-specific blockers can be varied as needed. The number of repeated hybridizations can be varied as needed. The different hybridizations can be done serially or in parallel or both serially and in parallel.

## Target Profiling

Another embodiment provides a method for obtaining a profile of one or more targets, for example nucleic acids such as miRNA present in a sample. An expression profile for one or more target nucleic acids can be obtained by interrogating a sample with an array including probes specific for one or more miRNAs. In some embodiments, the probes are less than about 23 nt and specifically hybridize to a target in a sequence dependent manner. A probe can bind to one or more regions of one target nucleic acid or can bind to the same region present in multiple targets. Detection of probe:target hybridization indicates the presence of a particular target or set of targets in the sample. The profile of a target can be correlated with pathologies such as cancer and the control of cell proliferation, cell death, fat metabolism, neuronal patterning, modulation of hematopoietic lineage differentiation, and control of leaf and flower development.

An exemplary array employed in the present method can include a plurality of features, each of which binds a target polynucleotide. For example, a feature can bind a gene or genomic nucleic acids corresponding to a gene, a transcript of the gene, a polynucleotide including a sequence from the gene, a fragment of a target gene or transcript, or a complement thereof. The feature can include an oligonucleotide, polynucleotide, polypeptide, or combinations thereof.

In an embodiment, the present method can include contacting such an array with a plurality of disclosed probes and arrays. The method can include contacting with a predetermined concentration or amount of at least one of the disclosed probes and arrays. The method can include contacting with a predetermined concentration or amount of each of the plurality of disclosed probes and arrays. The predetermined concentration or amount can be the same or different for each of the disclosed probes and arrays.

The plurality of disclosed probes and arrays can include disclosed probes and arrays for a plurality of the features on the array. In one embodiment, the plurality of disclosed probes and arrays includes at least one full length complement for at least one feature on the array. In another embodiment, the plurality of disclosed probes and arrays includes one disclosed probe and array for each unique feature on the array.

The plurality of disclosed probes and arrays can include disclosed probes and arrays for a plurality of genes of a cell, tissue, or organism. The plurality of disclosed probes and arrays can include a disclosed probes and arrays for each gene of a cell, tissue, or organism. The plurality of disclosed probes and arrays can include a disclosed probes and arrays for each of a subset of the genes of a cell, tissue, or organism. The subset of genes can include genes of interest, for example, with respect to development, disease, or disorder of the cell, tissue, or organism. At least one, or all, of the disclosed probes and arrays can include a detectable label, for example, a first detectable label.

The present method can include contacting the nucleic acid array with a sample suspected of containing a polynucleotide that can bind to a feature on the array. The target polynucleotide can be or include, for example, a gene, a transcript of the gene, a polynucleotide including a sequence from the gene, or a complement thereof. The method can include binding of the target polynucleotide to a nucleic acid in a feature at a location on the array. Monitoring for binding can employ an apparatus suitable for detecting binding that occurs. Should binding occur, the method can include detecting binding of the target polynucleotide to the array. The sample may include standardization molecules in addition to the targeted samples. The standardization molecules could be chosen miRNA, non-miRNA oligonucleotide sequences, or miRNA from another species than the target miRNA sample.

In an embodiment, the present method can include contacting the nucleic acid array with a sample including a plurality of target polynucleotides. At least one, or all, of the target polynucleotides can be capable of or suspected of being capable of binding to one or more features on the array. The plurality of target polynucleotides can each be from the same cell, tissue, or organism. The plurality of target polynucleotides can include polynucleotides of interest, for example, with respect to development, disease, or disorder of the cell, tissue, or organism. At least one, or all, of the target polynucleotides can include a detectable label.

In an embodiment, the present method can include reacting multiple arrays with standard mixtures or additions of target polynucleotides. The method can then include comparing the amount of binding detected at each corresponding location or feature on two or more of the arrays. Standardizing the arrays can be based on this comparison.

In an embodiment, the present method can include detecting a first detectable signal (e.g., colour) from the disclosed probes and arrays and a second detectable signal from a standard mixture of the target polynucleotides. The method can include comparing the strength of the first and second detectable signals. Quantitating the target polynucleotides in the sample can be based on this comparison.

Contacting can include any of a variety of known methods for contacting an array with a reagent, sample, or composition. For example, the method can include placing the array in a container and submersing the array in or covering the array with the reagent, sample, or composition. The method can include placing the array in a container and pouring, pipetting, or otherwise dispensing the reagent, sample, or composition onto features on the array. Alternatively, the method can include dispensing the reagent, sample, or composition onto features of the array, with the array being in or on any suitable rack, surface, or the like.

Detecting can include any of a variety of known methods for detecting a detectable signal from a feature or location of an array. Any of a variety of known, commercially available apparatus designed for detecting signals of or from an array can be employed in the present method. Such an apparatus or method can detect one or more of the detectable labels described herein below. For example, known and commercially available apparatus can detect colorimetric, fluorescent, or like detectable signals of an array. Surface plasmon resonance can be employed to detect binding of a disclosed probes and arrays to the array. The methods and systems for detecting a signal from a feature or location of an array can be employed for monitoring or scanning the array for any binding that occurs and results in a detectable signal. Monitoring or detecting can include viewing (e.g., visual inspection) of the array by a person.

The present disclosed probes and arrays or compositions can be provided in any variety of common formats. The present nucleotide or composition can be provided in a container, for example, as a solid (e.g., a lyophilized solid) or a liquid. In an embodiment, each of a plurality of disclosed probes and arrays is provided in its own container (e.g.,

vial, tube, or well). The present disclosed probes and arrays or compositions can be provided with materials for creating a nucleic acid array or with a complete nucleic acid array. In fact, the present polynucleotide or composition can be provided bound to one or more features of a nucleic acid array.

## Detectable Labels

The present disclosed probes, arrays or targets can include a detectable label, for example, a first detectable label. Sample polynucleotides can include a detectable label, for example, a second detectable label. Suitable labels include radioactive labels and non-radioactive labels, directly detectable and indirectly detectable labels, and the like. Directly detectable labels provide a directly detectable signal without interaction with one or more additional chemical agents. Suitable of directly detectable labels include colorimetric labels, fluorescent labels, and the like. Indirectly detectable labels interact with one or more additional members to provide a detectable signal. Suitable indirect labels include a ligand for a labeled antibody and the like.

Suitable fluorescent labels include any of the variety of fluorescent labels disclosed in United States Patent Application Publication No. 20010009762, the disclosure of which is incorporated herein by reference. Specific suitable fluorescent labels include: xanthene dyes, e.g., fluorescein and rhodamine dyes, such as fluorescein isothiocyanate (FITC), 6-carboxyfluorescein (commonly known by the abbreviations FAM and F), 6-carboxy-2',4',7',4,7-hexachlorofluorescein (HEX), 6-carboxy-4',5'-dichloro-2',7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G[5] or G[5]), 6-carboxyrhodamine-6G (R6G[6] or G[6]), and rhodamine 110; Alexa dyes, e.g. Alexa-fluor-547; cyanine dyes, e.g., Cy3, Cy5 and Cy7 dyes; coumarins, e.g., umbelliferone; benzimide dyes, e.g., Hoechst 33258; phenanthridine dyes, e.g., Texas Red; ethidium dyes; acridine dyes; carbazole dyes; phenoxazine dyes; porphyrin dyes; polymethine dyes, e.g., cyanine dyes such as Cy3, Cy5, etc; BODIPY dyes and quinoline dyes.

## Methods of Making Probes and Probe Compositions

The present disclosed probes and arrays can be produced by known methods. For example, the disclosed probes and arrays can be made by known methods for chemical synthesis. Chemical synthesis can employ commercial synthesizers. Chemical synthesis can produce thousands or more different sequences in multi-well plates. The method can include synthesizing segments of the polynucleotide and ligating the segments to form the polynucleotide. Chemical synthesis can be employed to make a transcript or fragment thereof.

The disclosed probes and arrays can be made by known recombinant methods. For example, conventional cloning and subcloning techniques and PCR can be employed to produce the disclosed probes and arrays. In an embodiment, targets or fragments such as exons can be isolated from biological samples by PCR.

## Recombinant Methods

DNA encoding genes or fragments of interest can be obtained from a suitable cDNA or genomic library. The libraries can be screened with probes designed to identify the exon of interest according to standard procedures. See, e.g., Sambrook et al., Molecular Cloning: A Laboratory Manual (New York: Cold Spring Harbor Laboratory Press, 2001). The probe can be labeled according to known methods so that it can be detected upon hybridization to DNA in the library. Alternatively, the probe may contain multiples of an miRNA binding region for a specific mRNA.

Host cells can be modified to produce the disclosed probes and arrays by transfecting or transforming with a cloning vector according to known techniques. The transfecting or transformed cells can be cultured in conventional nutrient media, which can be modified inducing promoters, selecting transformants, or amplifying the genes encoding the desired sequences. Suitable culture conditions (e.g., media, temperature, pH) can be chosen without undue experimentation. Suitable techniques for cell culture are known. See, e.g., Mammalian Cell Biotechnology: a Practical Approach, M. Butler, ed. (IRL Press, 1991) and Sambrook et al., *supra.*

Transfection can employ known methods appropriate for each host cell type. Suitable methods include $CaCl_2$, $CaPO_4$, liposome-mediated, or electroporation. Other known methods for introducing DNA into cells can be used. Suitable host cells for cloning or expressing DNA in vectors include prokaryote (e.g., *E. coli*), filamentous fungi or yeast, or higher eukaryote (e.g., insect or mammalian) cells. Such host cells are well-known and are publicly and even commercially available.

The DNA encoding the disclosed probes and arrays can be inserted into a replicable vector for amplification of the DNA. Suitable vectors are well-known and are publicly and even commercially available. The vector can, for example, be in the form of a plasmid, cosmid, virus particle, or phage. The appropriate nucleic acid sequence may be inserted into the vector by any of a variety of known procedures. The vector can also include, for example, a signal sequence, an origin of replication, a marker gene, an enhancer element, a promoter, and/or a transcription termination sequence. Such elements and sequences are known. The vector can be constructed employing well-known techniques. Gene amplification can be detected or measured in a host cell or other system by any of a variety of known methods (e.g., Southern blotting, Northern blotting, dot blotting, or in situ hybridization).

**Arrays**

Arrays on a substrate can be designed for testing against any type of sample, whether a trial sample, reference sample, a combination of them, or a known mixture of polynucleotides (in which latter case the arrays may be composed of features carrying unknown sequences to be evaluated). Any given substrate may carry one, two, four or more arrays disposed on a front surface of the substrate. Depending upon the use, any or all of the arrays may be the same or different from one another and each may contain multiple spots or features. An array may contain more than ten, more than one hundred, more than one thousand more ten thousand features, or even more than one hundred thousand features, in an area of less than 50 cm$^2$ 20 cm$^2$, or even less than 10 cm$^2$, or less than 1 cm$^2$. For example, features may have widths (that is, diameter, for a round spot) in the range from a 10 $\mu$m to 1.0 cm. In other embodiments each feature may have a width in the range of 1.0 $\mu$m to 1.0 mm, of 5.0 $\mu$m to 500 $\mu$m, or of 10 $\mu$m to 200 $\mu$m. Non-round features may have area ranges equivalent to that of circular features with the foregoing width (diameter) ranges. Feature sizes can be adjusted as desired, for example by using one or a desired number of pulses from a pulse jet to provide the desired final spot size.

Figure 2 shows an exemplary array system **200.** In this embodiment, probes **204** each have a melting temperature in a predetermined range, for example about 50°C to about 60°C for one or more target miRNAs **208.** Probes **204** are bound or attached to substrate **202** by a linker or stilt sequence **206.** For example, the 3' end of the probe can be bound to the linker or stilt sequence which in turn is bound to the substrate. The linker or stilt sequence can be a polymer that does not interact or hybridize with the one or more targets. Suitable linker or stilt sequence include, but are not limited to 6 to 12 nucleotides.

Substrates of the arrays can be any solid support, a colloid, gel or suspension. Exemplary solid supports include, but are not limited to metal, metal alloys, glass, natural polymers, non-natural polymers, plastic, elastomers, thermoplastics, pins, beads, fibers, membranes, or combinations thereof.

At least some, or all, of the features are of different compositions (for example, when any repeats of each feature composition are excluded the remaining features may account for at least 5%, 10%, or 20% of the total number of features), each feature can be of a homogeneous composition within the feature. Interfeature areas can (but not essentially) be present which do not carry any polynucleotide (or other biopolymer or chemical moiety of a type of which the features are composed). Such interfeature areas can be present where the arrays are formed by processes involving drop deposition of reagents but may not be present when, for example, photolithographic array fabrication processes are used. It will be appreciated though, that the interfeature areas, when present, could be of various sizes and configurations.

Array features will generally be arranged in a regular pattern (for example, rows and columns). However other arrangements of the features can be used where the user has, or is provided with, some means (for example, through an array identifier on the array substrate) of being able to ascertain at least information on the array layout (for example, any one or more of feature composition, location, size, performance characteristics in terms of significance in variations of binding patterns with different samples, or the like). Each array feature is generally of a homogeneous composition.

Each array may cover an area of less than 100 cm$^2$, or even less than 50 cm$^2$, 10 cm$^2$, or 1 cm$^2$. In many embodiments, the substrate carrying the one or more arrays will be shaped generally as a rectangular solid (although other shapes are possible), having a length of more than 4 mm and less than 1 m, for example, more than 4 mm and less than 600 mm, less than 400 mm, or less than 100 mm; a width of more than 4 mm and less than 1 m, for example, less than 500 mm, less than 400 mm, less than 100 mm, or 50 mm; and a thickness of more than 0.01 mm and less than 5.0 mm, for example, more than 0.1 mm and less than 2 mm, or more than 0.2 and less than 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse jets of either polynucleotide precursor units (such as monomers) in the case of in situ fabrication, or of externally synthesized polynucleotides. Such methods are described in detail in, for example, U.S. Patent Nos. 6,656,740; 6,458,583; 6,323,043; 6,372,483; 6,242,266; 6,232,072; 6,180,351; 6,171,797; or 6,323,043; or in U.S. Patent Application Serial No. 09/302,898 filed April 30, 1999 by Caren et al., and the references cited therein. These references are incorporated herein by reference. Other drop deposition methods can also be used for fabrication. Also, instead of drop deposition methods, known photolithographic array fabrication methods may be used. Interfeature areas need not be present particularly when the arrays are made by photolithographic methods as described in those patents.

## Methods Employing Arrays

Following receipt by a user of an array made by an apparatus or method of the present disclosure, it will can be exposed to a sample (for example, a fluorescently labeled polynucleotide or protein containing sample) in any well known manner and the array then read. Reading of the array may be accomplished by illuminating the array and reading the location and intensity of resulting fluorescence at multiple regions on each feature of the array. For example, a scanner may be used for this purpose which is similar to the AGILENT MICROARRAY SCANNER manufactured by Agilent Technologies, Palo Alto, CA. Other suitable apparatus and methods are described in U.S. patent applications: Serial No. 10/087447 "Reading Dry Chemical Arrays Through The Substrate" by Corson et al.; and in U.S. Patent Nos. 6,592,036; 6,583,424; 6,486,457; 6,406,849; 6,371,370; 6,355,921; 6,320,196; 6,251,685; and 6,222,664. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in U.S. Patent Nos. 6,251,685, or 6,221,583 and elsewhere). Data from read arrays may be processed in any known manner, such as described in U.S. Patent No. 6,591,196, U.S. Patent Application Serial No. 09/659,415 filed Sept. 11, 2000 for "Method And System For Extracting Data From Surface Array Deposited Features" (granted as US 6,768,820), and many commercially available array feature extraction software packages. A result obtained from the reading followed by a method of the present invention may be used in that form or may be further processed to generate a result such as that obtained by forming conclusions based on the pattern read from the array (such as whether or not a particular target sequence may have been present in the sample, or whether or not a pattern indicates a particular condition of an organism from which the sample came). A result of the reading (whether further processed or not) may be forwarded (such as by communication) to a remote location if desired, and received there for further use (such as further processing).

In one embodiment the array can be read using Matrix Assisted Laser Desorption Ionization Time-of-flight Mass Spectrometry (MALDI).

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

All publications and patent applications in this specification are indicative of the level of ordinary skill in the art to which this invention pertains.

The invention has been described with reference to various specific and preferred embodiments and techniques. However, it should be understood that many variations and modifications may be made while remaining within the scope of the invention.

The disclosures in United States patent application no. 11/173,590, from which this application claims priority, and in the abstract accompanying this application are incorporated herein by reference.

## Claims

1. A method for producing a probe set comprising:

    (a) determining the melting temperature of one or more probes for one or more target polynucleotides (102);
    (b) removing at least one base-pairing monomer from the one or more probes (104) to produce one or more truncated probes;
    (c) determining the melting temperature of the one or more truncated probes (106);
    (d) selecting, from among said truncated and untruncated probes, a set of probes (204) each having a melting temperature for the one or more target polynucleotides of within about 15° C of one another; and
    (e) combining the selected probes (108).

2. A method as claimed in claim 1, wherein the at least one base-pairing monomer is removed from a 3' region, a 5' region, or both 5' and 3' regions of the probe to produce one or more truncated probes.

3. A method as claimed in claim 1 or 2, wherein the combined probes and truncated probes have melting temperatures for the one or more target polynucleotides within about 10° C of one another.

**4.** A method as claimed in claim 1, 2 or 3, wherein the combined probes and truncated probes have melting temperatures for the one or more target polynucleotides within about 5° C of one another.

**5.** A method as claimed in any preceding claim, wherein the target polynucleotide is selected from the group consisting of miRNA, siRNA, tiny non-coding RNA, small modulatory RNA, mRNA, and fragments, modifications, and combinations thereof, and wherein the probes and truncated probes comprise DNA.

**6.** A method as claimed in any preceding claim, wherein the melting temperatures are determined empirically.

**7.** A method as claimed in any preceding claim, wherein the melting temperature of the one or more probes for one or more target polynucleotides is calculated and wherein the melting temperature of the one or more truncated probes is determined empirically.

**8.** A probe set obtainable by a method as claimed in any preceding claim.

**9.** An array (200) including the probe set of claim 8.

**10.** A method of obtaining a micro RNA (miRNA) profile of a sample comprising:

(a) contacting an array (200) with a sample, wherein the array consists of tethered probes (204) having a difference between a maximum melting temperature for one or more target miRNAs and a minimum melting temperature for the one or more target miRNAs of less than about 15° C; and
(b) detecting hybridization of miRNA (208) present in the sample with at least one probe.

# FIG. 1

100

102

determining the melting temperature of one or more probes for one or more target polynucleotides

104

removing at least one base - pairing nucleotide from the probes

106

determining the melting temperature of the one or more truncated probes

108

combining the probes having melting temperatures for the target polynucleotide of within about 15° C of one another

# FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5948902 A
- US 20010009762 A
- US 6656740 B
- US 6458583 B
- US 6323043 B
- US 6372483 B
- US 6242266 B
- US 6232072 B
- US 6180351 B
- US 6171797 B
- US 30289899 A, Caren
- US 087447 A
- US 6592036 B

- US 6583424 B
- US 6486457 B
- US 6406849 B
- US 6371370 B
- US 6355921 B
- US 6320196 B
- US 6251685 B
- US 6222664 B
- US 6221583 B
- US 6591196 B
- US 65941500 A
- US 6768820 B
- US 11173590 B

**Non-patent literature cited in the description**

- **ALTSCHUL et al.** *Nucleic Acids Res.,* 1997, vol. 25, 3389-3402
- **SAMBROOK ; RUSSELL.** *Molecular Cloning: A Laboratory Handbook,* 2001, 10.38-10.4110.47
- **WALLACE, R.B. et al.** Hybridization of synthetic oligodeoxyribonucleotides to phiX174 DNA: the effect of single base pair mismatch. *Nucleic Acids Res.,* 1979, vol. 6, 3543-3557

- **NICOLAS LE NOVERE.** MELTING, computing the melting temperature of nucleic acid duplex. *Bioinformatics,* 2001, vol. 17 (12), 1226-1227
- **SAMBROOK ; RUSSELL.** Molecular Cloning: A Laboratory Handbook. 2001, 10.38-10.41
- **SAMBROOK et al.** Molecular Cloning: A Laboratory Manual. Cold Spring Harbor Laboratory Press, 2001
- Mammalian Cell Biotechnology: a Practical Approach. IRL Press, 1991